# EUROPÄISCHE PATENTSCHRIFT

(11) **EP 0 471 287 B1**
(45) Veröffentlichungstag und Bekanntmachung des Hinweises auf die Patenterteilung: **21.06.1995**
(21) Anmeldenummer: 91113302.3
(22) Anmeldetag: 08.08.1991
(51) Int. Cl.: C07C 43/176, C07C 43/162, C07C 43/168, C07C 43/215, C07C 43/225, C07C 49/84, C07C 69/773, C07C 255/49, C09K 19/30

(54) **Alkyloxypropenyl Derivate**
Alkoxypropenyl derivatives
Dérivés d'alkoxypropènes

(30) Priorität: 15.08.1990 CH 2651/90
(43) Veröffentlichungstag der Anmeldung: 19.02.1992
(73) Patentinhaber: F. HOFFMANN-LA ROCHE AG, 4002 Basel (CH)
(72) Erfinder: Kelly, Stephen, CH-4313 Möhlin (CH)
(74) Vertreter: Cottong, Norbert A.

(56) Entgegenhaltungen:
- WO-A-89/02884
- WO-A-89/08633
- WO-A-89/08687
- WO-A-89/08689
- WO-A-90/01021
- DE-A- 3 714 043
- DE-A- 3 807 957
- DE-A- 3 904 817
- DE-A- 3 909 802
- DE-A- 4 027 840

## Beschreibung

Die vorliegende Erfindung betrifft neue Verbindungen mit endständigem Alkoxypropenylrest, deren Herstellung, flüssigkristalline Gemische, die solche Verbindungen enthalten sowie die Verwendung für elektro-optische Zwecke.

Flüssige Kristalle werden vor allem als Dielektrika in Anzeigevorrichtungen verwendet, da die optischen Eigenschaften solcher Substanzen durch eine angelegte Spannung beeinflusst werden können. Solche Anzeigevorrichtungen sind dem Fachmann bestens bekannt, es sind dies beispielsweise Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("super twisted nematic"), SBE-Zellen ("super-birefringence effect") und OMI-Zellen ("optical mode interference"). Die gebräuchlichsten Anzeigevorrichtungen beruhen auf dem Schadt-Helfrich-Effekt und besitzen eine verdrillt nematische Struktur.

Die Flüssigkristallmaterialien sollten eine gute chemische und thermische Stabilität haben und ausserdem gegenüber elektrischen Feldern und elektromagnetischen Strahlungen stabil sein. Sie sollten bei betriebsüblichen Temperaturen eine geeignete Mesophase besitzen, beispielsweise eine nematische, cholesterische oder getiltete smektische Phase. Die Flüssigkristallmaterialien sollten ferner niedere Viskosität aufweisen, in den Zellen kurze Ansprechzeiten, tiefe Schwellenspannungen und einen hohen Kontrast ergeben.

Weitere Eigenschaften, wie die elektrische Leitfähigkeit, die dielektrische Anisotropie und die optische Anisotropie, müssen je nach Zellentyp und Anwendungsgebiet unterschiedlichen Anforderungen genügen.

Beispielsweise sollten Materialien für Zellen mit verdrillt nematischer Struktur eine positive dielektrische Anisotropie und eine möglichst geringe elektrische Leitfähigkeit aufweisen. Neben dem generellen Interesse an Flüssigkristallmaterialien mit hoher optischer Anisotropie besteht in letzter Zeit ein vermehrtes Interesse an Materialien mit niedriger optischer Anisotropie, insbesondere für aktiv adressierte Flüssigkristallanzeigen, z.B. bei TFT-Anwendungen ("thin film transistor") in Fernsehgeräten. Flüssigkristalline Verbindungen, welche sich für obige Zwecke eignen, sind bekannt. So sind beispielsweise in DE-A-3 909 802 und WO 89/02884 Verbindungen mit einem Difluormethylphenyl-, Difluormethoxyphenyl- und Trifluormethoxyphenyl-Rest beschrieben. Ausserdem sind flüssigkristalline 2,3-Difluorphenyl-Derivate mit negativer dielektrischer Anisotropie (Δε) aus WO 90/01021, DE-A-3 807 957, WO 89/08633 und WO 89/08689 bekannt.

Flüssigkristalle werden zwecks Optimierung der Eigenschaften in der Regel als Mischungen mehrerer Komponenten verwendet. Es ist daher wichtig, dass die Komponenten untereinander gut mischbar sind. Cholesterische Gemische können vorzugsweise aus einem oder mehreren optisch aktiven Dotierstoffen und einem nematischen Flüssigkristallmaterial bestehen. Solche Mischungen werden beispielsweise in DE-A-3 714 043 beschrieben.

Gegenstand der vorliegenden Erfindung sind Verbindungen der allgemeinen Formel 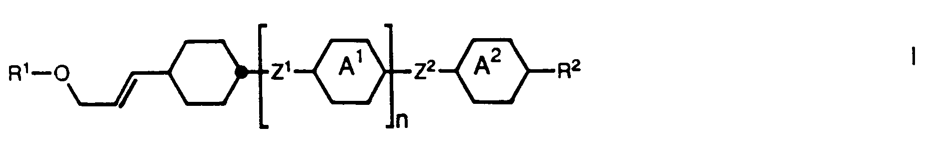
worin die Ringe A¹ und A² unabhängig voneinander unsubstituiertes oder mit Halogen substituiertes 1,4-Phenylen, in welchem falls unsubstituiert gegebenenfalls 1 oder 2 CH-Gruppen durch Stickstoff ersetzt sind, oder trans-1,4-Cyclohexylen oder trans-1,3-Dioxan-2,5-diyl darstellen; n entweder 0 oder 1 ist; Z¹ und Z² unabhängig voneinander eine einfache Kovalenzbindung oder -CH₂CH₂-, -COO-, -OOC-, -OCH₂-, -CH₂O-, -(CH₂)₄-, -O(CH₂)₃-, -(CH₂)₃O-, die trans-Form von -O-CH₂-CH=CH-, -CH=CH-CH₂-O-, -(CH₂)₂CH=CH- oder -CH=CH-(CH₂)₂- bedeuten; R¹ eine Alkylgruppe bezeichnet; R² Halogen, Cyano, Trifluoracetyl, gegebenenfalls mit Fluor substituiertes Alkyl mit 1 bis 12 Kohlenstoffatomen, in welchem gegebenenfalls 1 oder 2 nicht benachbarte -CH₂-Gruppen durch Sauerstoff und/oder eine -CH₂-CH₂- Gruppe durch -CH=CH- ersetzt sind, darstellt.

Es wurde gefunden, dass die Einführung einer Alkoxyalkenylgruppe die Tendenz zur Ausbildung von Flüssigkristallphasen und die Ansprechzeiten günstig beeinflusst. Die dielektrische Anisotropie (Δε) und die optische Anisotropie (Δn) können je nach Wahl der Ringe und Substituenten variiert werden, so haben beispielsweise Verbindungen der Formel I, worin R² Cyano und Z² -COO- bedeutet, eine hohe positive dielektrische Anisotropie; Verbindungen der Formel I, worin die Ringe A¹ und A² 1,4-Phenylen, in welchem gegebenenfalls eine oder zwei CH-Gruppen mit Stickstoff ersetzt sind, haben eine hohe optische Anisotropie.

Der Ausdruck ''unsubstituiertes oder mit Halogen substituiertes 1,4-Phenylen, in welchem gegebenenfalls 1 oder 2 CH-Gruppen durch Stickstoff ersetzt sind'' umfasst in der vorliegenden Erfindung Gruppen wie 1,4-Phenylen, 2-Fluor-1,4-phenylen, 2,3-Difluor-1,4-phenylen, 2-Chlor-1,4-phenylen, 2-Brom-1,4-phenylen, Pyridin-2,5-diyl, Pyrimidin-2,5-diyl und dergleichen. Bevorzugte Gruppen sind 1,4-Phenylen, 2-Fluor-1,4-phenylen, 2,3-Difluor-1,4-phenylen, Pyridin-2,5-diyl und Pyrimidin-2,5-diyl.

Der Ausdruck ''Halogen'' bezeichnet Chlor, Fluor, Brom und Jod, insbesondere Chlor und Fluor.

Der Ausdruck "gegebenenfalls mit Fluor substituiertes Alkyl mit 1 bis 12 Kohlenstoffatomen, bei welchem gegebenenfalls 1 oder 2 nicht benachbarte CH₂-Gruppen durch Sauerstoff und/oder eine -CH₂-CH₂- Gruppe durch -CH=CH- ersetzt sind" bezeichnet im Rahmen der vorliegenden Erfindung geradkettige und verzweigte (gegebenenfalls chirale) Reste wie Alkyl, 1E-Alkenyl, 3E-Alkenyl, 4-Alkenyl, Alkenyl mit endständiger Doppelbindung, Alkoxy, Alkenyloxy mit endständiger Doppelbindung, Alkoxyalkyl, 2E-Alkenyloxy, 3-Alkenyloxy, Alkenyloxyalkyl, Haloalkyl, Haloalkoxy und dergleichen. Beispiele bevorzugter Reste sind Methyl, Aethyl, Propyl, Butyl, Pentyl, Hexyl, Heptyl, Octyl, Nonyl, Decyl, Undecyl, Dodecyl, Vinyl, 1E-Propenyl, 1E-Butenyl, 1E-Pentenyl, 1E-Hexenyl, 1E-Heptenyl, 3-Butenyl, 3E-Pentenyl, 3E-Hexenyl, 3E-Heptenyl, 4-Pentenyl, 4Z-Hexenyl, 4Z-Heptenyl, 5-Hexenyl, 6-Heptenyl, 7-Octenyl, 8-Nonenyl, 9-Decenyl, 10-Undecenyl, 11-Dodecenyl, Methoxy, Aethoxy, Propyloxy, Butyloxy, Pentyloxy, Hexyloxy, Heptyloxy, Octyloxy, Nonyloxy, Decyloxy, Undecyloxy, Dodecyloxy, Allyloxy, 2E-Butenyloxy, 2E-Pentenyloxy, 2E-Hexenyloxy, 2E-Heptenyloxy, 3-Butenyloxy, 3Z-Pentenyloxy, 3Z-Hexenyloxy, 3Z-Heptenyloxy, 4-Pentenyloxy, 5-Hexenyloxy, 6-Heptenyloxy, 7-Octenyloxy, 8-Nonenyloxy, 9-Decenyloxy, 10-Undecenyloxy, 11-Dodecenyloxy, Methoxymethyl, Aethoxymethyl, Propyloxymethyl, Fluormethyl, Difluormethyl, Trifluormethyl, Fluormethoxy, Difluormethoxy und Trifluormethoxy.

Der Ausdruck "Alkyl" umfasst in der vorliegenden Erfindung einen geradkettigen oder verzweigten Alkylrest mit 1-12 Kohlenstoffatomen.

Ein bevorzugter Aspekt der Erfindung betrifft Verbindungen der allgemeinen Formel I, worin R¹ einen geradkettigen Alkylrest mit 1 bis 7, vorzugsweise 1 bis 3 Kohlenstoffen oder verzweigten, gegebenenfalls chiralen Alkylrest mit 3 bis 7 Kohlenstoffatomen darstellt. Ganz besonders bevorzugt werden die Verbindungen der allgemeinen Formel I, worin R¹ Methyl, Aethyl oder Propyl bedeutet.

Ein bevorzugter Aspekt der Erfindung betrifft Verbindungen der Formel I, in welchen eine der Gruppen Z¹ und Z² eine einfache Kovalenzbindung bedeutet.

Eine bevorzugte Gruppe Verbindungen der Formel I umfasst Verbindungen der allgemeinen Formel 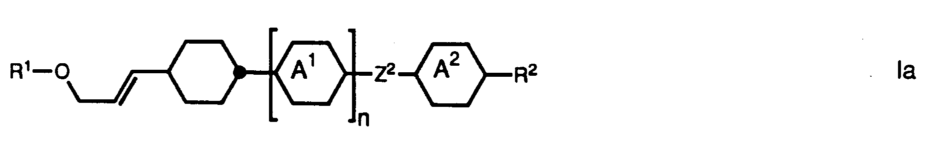
worin Ring A¹ 1,4-Phenylen oder trans-1,4-Cyclohexylen bedeutet; n entweder 0 oder 1 ist; Ring A² unsubstituiertes oder mit Halogen substituiertes 1,4-Phenylen bedeutet; R¹, R² und Z² die in Formel I angegebene Bedeutung haben.

Ein bevorzugter Aspekt der Erfindung betrifft Verbindungen der Formel Ia, worin Z² eine einfache Kovalenzbindung, -CH₂CH₂-, -OOC-, -COO-, -OCH₂-oder -CH₂O- darstellt.

Die Eigenschaften der Verbindungen Ia können je nach Zahl und Bedeutung der Ringe und Substituenten in breiten Bereichen variiert werden.

Besonders bevorzugte Verbindungen der Formel Ia sind Verbindungen der allgemeinen Formel 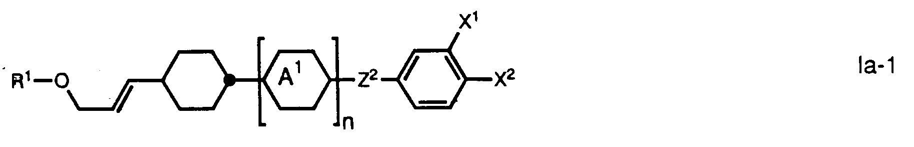
worin R¹ die in Formel I angegebene Bedeutung hat; n, Z² und Ring A¹ die in Formel Ia angegebenen Bedeutungen haben; X¹ Wasserstoff, Chlor oder Fluor bedeutet; und X² Fluor oder Chlor bezeichnet.

Vorzugsweise stellt Z² in der Formel Ia-1 eine einfache Kovalenzbindung oder -CH₂CH₂- dar. Die Verbindungen der Formel Ia-1 eignen sich beispielsweise dank ihrer niedrigen Schwellenspannung für die Anwendung in Flüssigkristallmaterialien, welche in TFT-Zellen verwendet werden.

Besonders bevorzugte Verbindungen der Formel Ia sind ferner Verbindungen der allgemeinen Formel 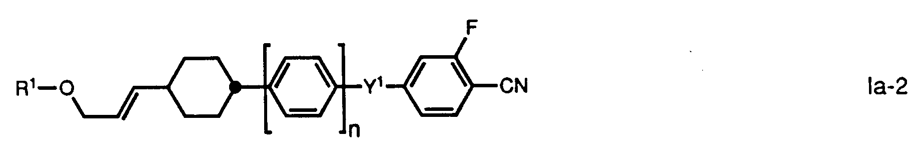
worin R¹ die in Formel I angegebene Bedeutung hat; n entweder 0 oder 1 ist; Y¹ eine einfache Kovalenzbindung oder -CH₂CH₂-, -COO-, -OOC-, -OCH₂-, -CH₂O-, -(CH₂)₄-, -O(CH₂)₃-, -(CH₂)₃O-, die trans-Form von -O-CH₂-CH=CH-, -CH=CH-CH₂-O-, -(CH₂)₂CH=CH- oder -CH=CH-(CH₂)₂- bedeutet.

Vorzugsweise bedeutet Y¹ in der Formel Ia-2 eine einfache Kovalenzbindung -CH₂CH₂- oder -COO-. Die Verbindungen der Formel Ia-2 zeichnen sich beispielsweise durch vergleichsweise hohe dielektrische Anisotropie und niedrige Schwellenspannung aus.

Weitere bevorzugte Verbindungen der Formel Ia sind Verbindungen der allgemeinen Formel 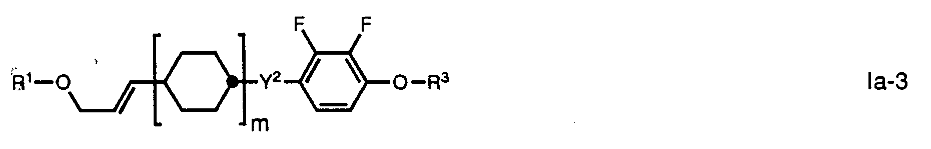
worin R¹ die in Formel I angegebene Bedeutung hat; m entweder 1 oder 2 ist; Y² eine einfache Kovalenzbindung oder -CH₂CH₂-, -COO-, -OOC-, -OCH₂-, -CH₂O-, -(CH₂)₄-, -O(CH₂)₃-, -(CH₂)₃O-, die trans-Form von -O-CH₂-CH=CH-, -CH=CH-CH₂-O-, -(CH₂)₂CH=CH- oder -CH=CH-(CH₂)₂- bedeutet; und R³ eine Alkylgruppe mit 1-12 Kohlenstoffatomen, in welcher gegebenenfalls -CH₂-CH₂- durch -CH=CH- ersetzt ist, oder eine Halo-Alkylgruppe vorzugsweise Fluormethyl, Difluormethyl oder Trifluormethyl bedeutet.

Vorzugsweise bedeutet Y² in der Formel Ia-3 eine einfache Kovalenzbindung, -CH₂CH2- oder -CH₂O-. Die Verbindungen der Formel Ia-3 können beispielsweise wegen ihrer negativen dielektrischen Anisotropie für Anwendung in DAP-Zellen (deformation of aligned phases) oder ECB-Zellen (electrically controlled birefringence), verwendet werden.

Eine weitere Gruppe bevorzugter Verbindungen der allgemeinen Formel I umfasst Verbindungen der allgemeinen Formel 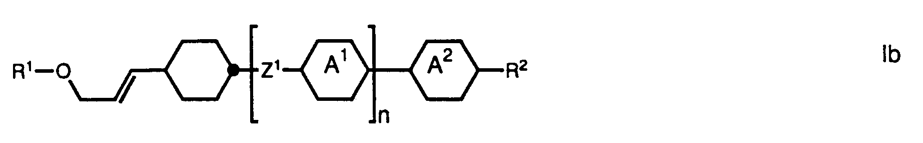
worin R¹ die in Formel I angegebene Bedeutung hat; Z¹ die in Formel I angegebenen Bedeutungen hat, vorzugsweise eine einfache Kovalenzbindung, -CH₂CH₂- oder -COO- darstellt; n entweder 0 oder 1 ist; R² und die Ringe A¹ und A² die in Formel I angegebenen Bedeutungen haben.

Besonders bevorzugte Aspekte der vorliegenden Erfindung sind Verbindungen der allgemeinen Formel Ib, worin der Ring A¹ 1,4-Phenylen und der Ring A² unsubstituiertes oder substituiertes 1,4-Phenylen bedeutet.

Die Verbindungen der Formel Ib eignen sich beispielsweise für ferroelektrische Anwendungen, besonders bevorzugte Verbindungen der Formel Ib sind 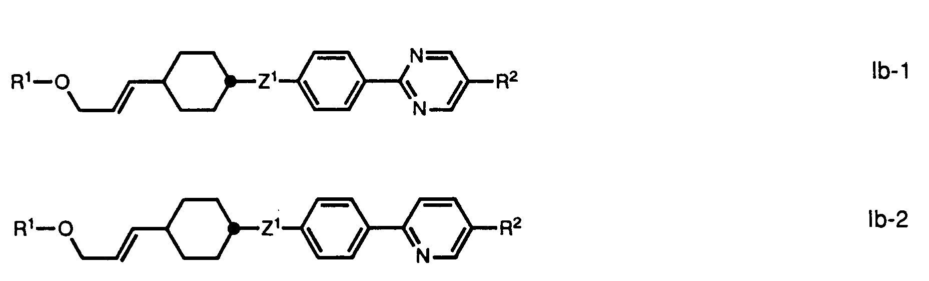
worin R¹ und R² die in Formel I gegebenen Bedeutungen haben; und Z¹ die in Formel I gegebene Bedeutung hat, vorzugsweise eine einfache Kovalenzbindung oder -COO- bezeichnet.

Ein besonders bevorzugter Aspekt der Erfindung umfasst Verbindungen der Formeln I, Ia, Ia-1 bis 3 und Ib, Ib-1 und 2, worin R¹ Methyl, Aethyl oder Propyl bedeutet.

Die vorliegende Erfindung bietet somit eine breite Palette neuer Komponenten zur weiteren Optimierung und Modifizierung von Flüssigkristallmaterialien.

Die Verbindungen der allgemeinen Formel I können erfindungsgemäss auf an sich bekannte Weise, beispielsweise nach den in Schema 1 und 2, sowie nach den in den Beispielen illustrierten Methoden, hergestellt werden.

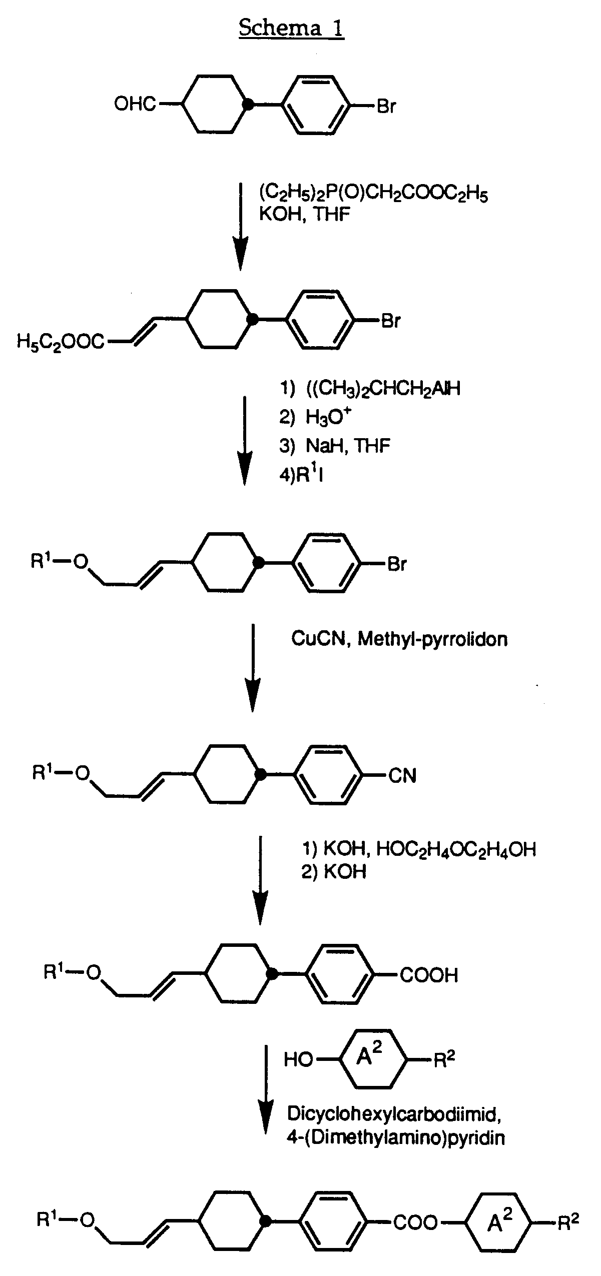

Die Verbindungen der Formel I können in Form von Gemischen untereinander und/oder mit anderen Flüssigkristallkomponenten verwendet werden.

Die erfindungsgemässen flüssigkristallinen Gemische enthalten mindestens zwei Komponenten, wovon mindestens eine Komponente eine Verbindung der Formel I ist. Eine zweite und gegebenenfalls weitere Komponenten können weitere Verbindungen der Formel I oder andere Flüssigkristallkomponenten sein.

Aufgrund der guten Löslichkeit der Verbindungen der Formel I und aufgrund ihrer guten Mischbarkeit untereinander kann ihr Anteil in den erfindungsgemässen Gemischen relativ hoch sein. Im allgemeinen ist jedoch ein Anteil von etwa 1-50 Gew.-%, insbesondere etwa 5-30 Gew.-% an Verbindungen der Formel I bevorzugt.

Vorzugsweise enthalten die erfindungsgemässen Gemische neben einer oder mehreren Verbindungen der Formel I eine oder mehrere Verbindungen aus der Gruppe der Verbindungen der allgemeinen Formel 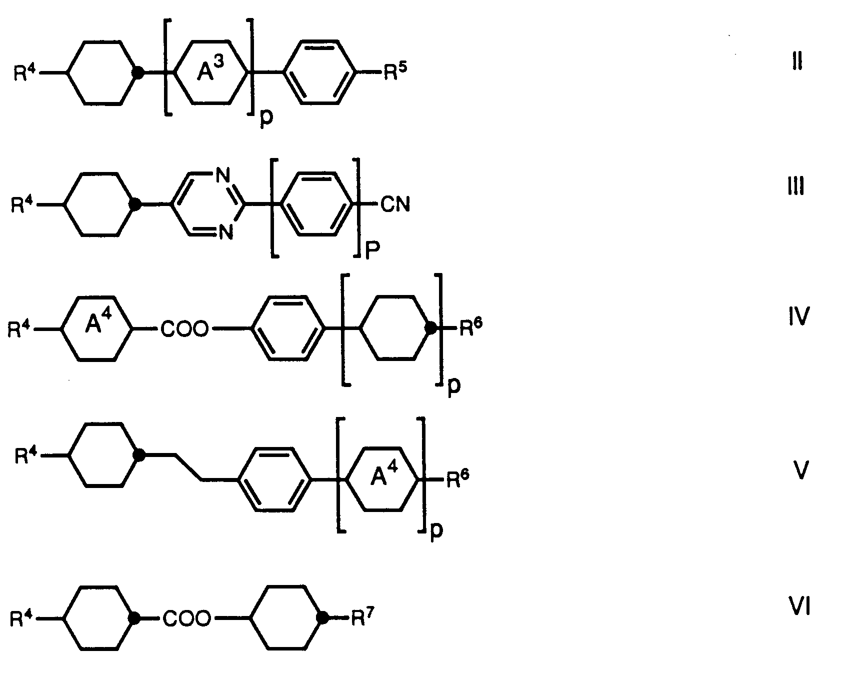 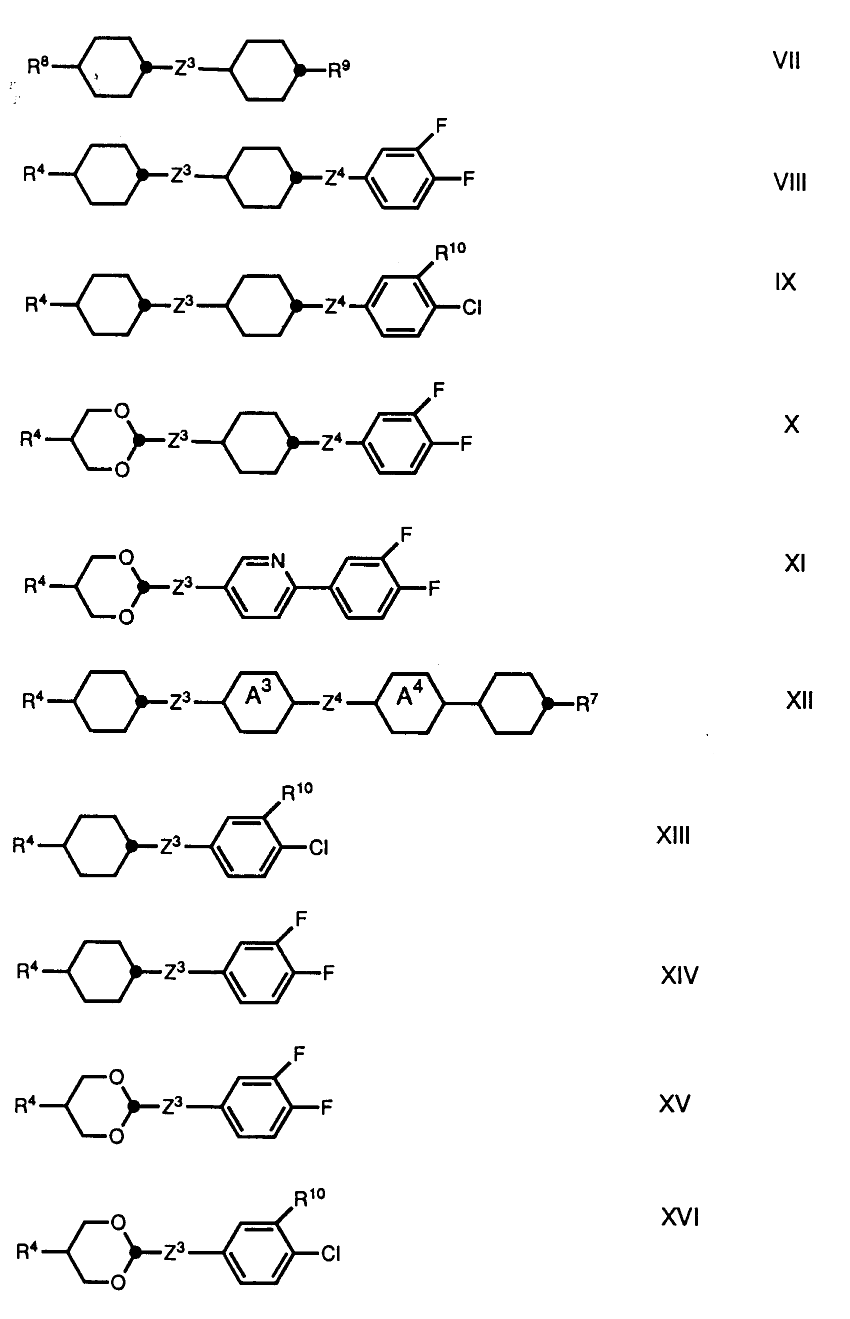 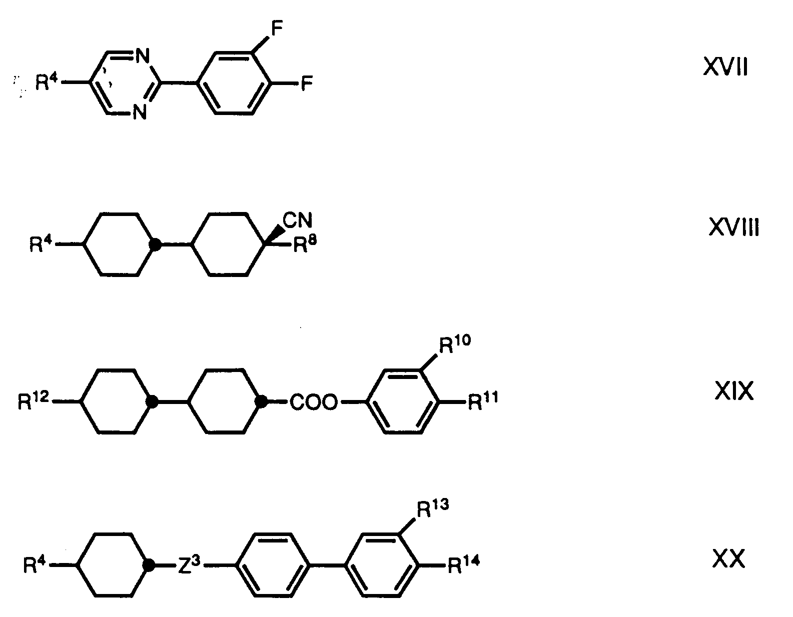
worin p für die Zahl 0 oder 1 steht; R⁴ und R⁷ unabhängig voneinander Alkyl, 3E-Alkenyl, 4-Alkenyl oder an einem gesättigten Ring auch 1E-Alkenyl bedeutet; Ring A³ 1,4-Phenylen, trans-1,4-Cyclohexylen, Pyrimidin-2,5-diyl oder trans-1,3-Dioxan-2,5-diyl darstellt; R⁵ Cyano, -NCS, Fluor, Alkyl, 3E-Alkenyl, 4-Alkenyl, Alkoxy, 2E-Alkenyloxy oder 3-Alkenyloxy bezeichnet; Ring A⁴ 1,4-Phenylen oder trans-1,4-Cyclohexylen darstellt; R⁶ Alkyl, 3E-Alkenyl, 4-Alkenyl oder an einem Cyclohexanring auch 1E-Alkenyl oder an einem Benzolring auch Cyano, -NCS, Alkoxy, 2E-Alkenyloxy oder 3-Alkenyloxy bedeutet; R⁸ Alkyl, 1E-Alkenyl, 3E-Alkenyl oder 4-Alkenyl bezeichnet; Z³ eine einfache Kovalenzbindung oder -CH₂CH₂- darstellt; R⁹ Cyano, Alkyl, 1E-Alkenyl, 3E-Alkenyl, 4-Alkenyl, Alkoxy, 2E-Alkenyloxy, 3-Alkenyloxy, Alkoxymethyl oder (2E-Alkenyl)oxymethyl bedeutet; R¹⁰ Wasserstoff, Fluor oder Chlor bedeutet; R¹¹ Fluor, Wasserstoff oder Cyano darstellt; R¹² für Alkyl-, 1E-Alkenyl, 3E-Alkenyl oder 4-Alkenyl steht; R¹³ Wasserstoff oder Fluor bedeutet; und R¹⁴ Fluor oder Chlor darstellt.

Der obige Ausdruck "gesättigter Ring" umfasst trans-1,4-Cyclohexylen und trans-1,3-Dioxan-2,5-diyl. Reste R⁴ bis R⁹ besitzen vorzugsweise höchstens je 12 Kohlenstoffatome, besonders bevorzugt höchstens je 7 Kohlenstoffatome. Geradkettige Reste sind im allgemeinen bevorzugt. Die Verbindungen mit den allgemeinen Formeln II-XX sind bekannt und zum Teil im Handel erhältlich.

Die Herstellung der flüssigkristallinen Gemische und der elektrooptischen Vorrichtungen kann in an sich bekannter Weise erfolgen.

Die Erfindung wird durch die folgenden Beispiele weiter veranschaulicht. In den Beispielen bedeutet C eine kristalline, N eine nematische, S eine smektische und I die isotrope Phase. V₁₀ bezeichnet die Spannung für 10% Transmission. tₒₙ und t_{off} bezeichnen die Einschaltzeit bzw. die Ausschaltzeit. Δn bezeichnet die optische Anisotropie.

### Beispiel 1

Ein Gemisch von 0,5 g Natriumhydrid und 50 ml Tetrahydrofuran wurde unter Stickstoffbegasung mit 2,6 g 1-Brom-4-[trans-4-[(E)-3-hydroxypropenyl]cyclohexyl]benzol versetzt, 2 Stunden gerührt, mit 1,6 g Methyljodid versetzt und anschliessend über Nacht bei Raumtemperatur gerührt. Das Reaktionsgemisch wurde mit 500 ml Wasser versetzt und viermal mit je 50 ml Hexan extrahiert, die vereinigten organischen Phasen wurden zweimal mit je 500 ml Wasser gewaschen, über Magnesiumsulfat getrocknet, die Suspension filtriert und das Filtrat eingeengt. Der Rückstand wurde an Kieselgel mit Hexan chromatographisch gereinigt und das erhaltene Produkt aus Hexan bei -78°C umkristallisiert, dies ergab 2,4 g 1-Brom-4-[trans-4-[(E)-3-methoxypropenyl]cyclohexyl]benzol, Smp. (C-I) 47°C.

Das als Ausgangsmaterial verwendete 1-Brom-4-[trans-4-[(E)-3-hydroxypropenyl)cyclohexyl]benzol wurde wie folgt hergestellt:
a) In einem Sulfierkolben mit Thermometer, mechanischem Rührer, Tropftrichter und Festsubstanz-Zugaberohr wurden unter Argonbegasung 20,4g Triphenyl-methoxymethyl-phosphoniumchlorid in 60 ml t-Butylmethyläther aufgeschlärnmt und bei -10°C innert 10 Minuten mit 6,6 g festem Kalium-t-butylat versetzt. Nach beendeter Zugabe wurde während 30 Minuten bei -10°C bis 0°C gerührt, dann das tieforange, heterogene Reaktionsgemisch bei 0°G tropfenweise mit einer Lösung von 10 g 4-(4-Bromphenyl)cyclohexanon in 50 ml absolutem Tetrahydrofuran versetzt, anschliessend 24 Stunden bei Raumtemperatur gerührt, auf 500 ml Hexan gegossen und filtriert. Niederdruckchromatographie des eingeengten Rückstandes (13,9 g) an Kieselgel mit Hexan/Aethylacetat (Vol. 9:1) ergab 10,4 g 1-Brom-4-[4-(methoxymethyliden)cyclohexyl]benzol als farbloses Oel.
b) In einem Rundkolben wurde ein Gemisch von 10,4 g 1-Brom-4-[4-(methoxymethylen)cyclohexyl]benzol und 100 ml Tetrahydrofuran/2N Salzsäure (Vol. 4:1) während 30 Minuten zum Rückfluss erhitzt. Anschliessend wurde das Reaktionsgemisch auf 100 ml Wasser gegossen und dreimal mit je 100 ml Diäthyläther extrahiert. Die organischen Phasen wurden mit 100 ml Wasser gewaschen, über Magnesiumsulfat getrocknet und eingeengt. Es resultierten 9,4 g 4-(4-Bromphenyl)cyclohexancarboxaldehyd als weisse Kristalle; trans/cis-Verhältnis ca. 5:3.
c) In einem Sulfierkolben wurde unter Argonbegasung ein Gemisch von 9,1 g 4-(4-Bromphenyl)cyclohexancarboxaldehyd und 3,8 g gemahlenem Kaliumhydroxid in 200 ml Tetrahydrofuran bei Raumtemperatur vorgelegt und innert 15 Minuten mit 9,2 g Triäthylphosphonoacetat versetzt, über Nacht gerührt, in 100 ml Wasser aufgenommen und dreimal mit je 100 ml Methylenchlorid extrahiert. Die organischen Phasen wurden zweimal mit je 100 ml Wasser gewaschen, über Magnesiumsulfat getrocknet und eingeengt. Niederdruckchromatographie des Rückstandes (12 g) an Kieselgel mit Hexan/Aethylacetat (Vol. 9:1) ergab 11,7 g (E)-3-[trans-4-(4-Bromphenyl)cyclohexyl]acrylsäure-äthylester als farblose Kristalle.
d) In einem Sulfierkolben mit Thermometer und Serumkappe wurde unter Argonbegasung eine Lösung von 12,7 g (E)-3-[trans-4-(4-Bromphenyl)cyclohexyl]acrylsäure-äthylester in 225 ml Toluol bei 0°C vorgelegt und innert 10 Minuten mit 47,0 ml einer 1,2M Lösung von Diisobutylaluminiumhydrid in Toluol versetzt. Nach beendeter Zugabe wurde das Reaktionsgemisch über Nacht bei Raumtemperatur gerührt, bevor es auf 100 ml 1,0N Schwefelsäure gegossen und zweimal mit je 50 ml Aethylacetat extrahiert wurde. Die organischen Phasen wurden mit 50 ml Wasser gewaschen, über Magnesiumsulfat getrocknet und eingeengt. Der Rückstand (10,9 g) wurde an Kieselgel mit Hexan/Aethylacetat (Vol. 7:3) chromatographisch gereinigt. Das erhaltene 1-Brom-4-[trans-4-[(E)-3-hydroxypropenyl)cyclohexyl]brombenzol wurde zweimal aus Cyclohexan umkristallisiert. Dies ergab 2,6 g reines Produkt mit Smp. 104°C.

In analoger Weise können folgende Verbindungen hergestellt werden:
1-Fluor-4-[trans-4-[(E)-3-methoxypropenyl]cyclohexyl]-benzol, Smp. (C-I) 27°C;
1-Chlor-4-[trans-4-[(E)-3-methoxypropenyl]cyclohexyl]benzol, Smp. (C-I) 40°C;
1-Brom-4-[trans-4-[(E)-3-methoxypropenyl]cyclohexyl]benzol, Smp. (C-I) 47°C;
1-Chlor-2-fluor-4-[trans-4-[(E)3-methoxypropenyl]cyclohexyl]benzol, Smp. (C-I) 23°C;
1,2-Difluor-4-[trans-4-[(E)3-methoxypropenyl]cyclohexyl]benzol, Smp. (C-I) 24°C;
1-Methyl-4-[trans-4-[(E)-3-methoxypropenyl]cyclohexyl]benzol;
1-Aethyl-4-[trans-4-[(E)-3-methoxypropenyl]cyclohexyl]benzol;
1-Propyl-4-[trans-4-[(E)-3-methoxypropenyl]cyclohexyl]benzol;
1-Methoxy-4-[trans-4-[(E)-3-methoxypropenyl]cyclohexyl]benzol, Smp. (C-I) 24°C, Klp. (N-I) (17°C);
1-Aethoxy-4-[trans-4-[(E)-3-methoxypropenyl]cyclohexyl]benzol, Smp. (C-N) 19°C, Klp. (N-I) 46°C;
1-Propyloxy-4-[trans-4-[(E)-3-methoxypropenyl]cyclohexyl]benzol, Smp. (C-I) 30°C, Klp. (N-I) (19°C);
1-Butyloxy-4-[trans-4-[(E)-3-methoxypropenyl]cyclohexyl]benzol, Smp. (C-N) 23°C, Klp. (N-I) 40°C;
1-Pentyloxy-4-[trans-4-[(E)-3-methoxypropenyl]cyclohexyl]benzol, Smp. (C-N) 30°C, Klp. (N-I) 31°C;
1-Hexyloxy-4-[trans-4-[(E)-3-methoxypropenyl]cyclohexyl]benzol, Smp. (C-S_{B}) 16°C, S_{B}-N 30°C, Klp. (N-I) 38°C;
1-Heptyloxy-4-[trans-4-[(E)-3-methoxypropenyl]cyclohexyl]benzol, Smp. (C-S_{B}) 14°C, Klp. (S_{B}-I) 38°C;
1-(Trifluormethyl)-4-[trans-4-[(E)-3-methoxypropenyl]cyclohexyl]benzol;
1-(Trifluormethoxy)-4-[trans-4-[(E)-3-methoxypropenyl]cyclohexyl]benzol;
1-(Difluormethoxy)-4-[trans-4-[(E)-3-methoxypropenyl]cyclohexyl]benzol;
1-Fluor-4-[trans-4-(trans-4-[(E)-3-methoxypropenyl]cyclohexyl)cyclohexyl]benzol, Smp. (C-N) 83°C, Klp. (N-I) 170°C;
1-Chlor-4-[trans-(trans-4-[(E)-3-methoxypropenyl]cyclohexyl)cyclohexyl]benzol;
1-Brom-4-[trans-4-(trans-4-[(E)-3-methoxypropenyl]cyclohexyl)cyclohexyl]benzol, Smp. (C-N) 120°C, Klp. (N-I) 207°C;
1-Chlor-2-fluor-4-[trans-4-(trans-4-[(E)-3-methoxypropenyl]cyclohexyl)cyclohexyl]benzol, Smp. (C-N) 69°C, Klp. (N-I) 168°C;
1-(Trifluoracetyl)-4-[trans-4-(trans-4-[(E)-3-methoxypropenyl]cyclohexyl)cyclohexyl]benzol;
1,2-Difluor-4-[trans-4-(trans-4-[(E)-3-methoxypropenyl]cyclohexyl)cyclohexyl]benzol, Smp. (C-N) 47°C, Klp. (N-I) 133°C;
1-Methyl-4-[trans-4-(trans-4-[(E)-3-methoxypropenyl]cyclohexyl)cyclohexyl]benzol, Smp. (C-S_{B}) 79°C, S_{B}-N 99°C, Klp. (N-I) 193°C;
1-Aethyl-4-[trans-4-(trans-4-[(E)-3-methoxypropenyl]cycloexyl)cyclohexyl]benzol;
1-Propyl-4-[trans-4-(trans-4-[(E)-3-methoxypropenyl]cycloexyl)cyclohexyl]benzol;
1-Methyl-2-fluor-4-[trans-4-(trans-4-[(E)-3-methoxypropenyl]cyclohexyl)cyclohexyl]benzol, Smp. (C-N) 58°C, Klp. (N-I) 160°C;
1-(Trifluormethyl)-4-[trans-4-(trans-4-[(E)-3-methoxypropenyl]cyclohexyl)cyclohexyl]benzol;
1-(Trifluormethoxy)-4-[trans-4-(trans-4-[(E)-3-methoxypropenyl]cyclohexyl)cyclohexyl]benzol;
1-(Difluormethoxy)-4-[trans-4-(trans-4-[(E)-3-methoxypropenyl]cyclohexyl)cyclohexyl]benzol;
1-(Trifluoracetyl)-4-[trans-4-(trans-4-[(E)-3-methoxypropenyl]cyclohexyl)cyclohexyl]benzol;
1-Fluor-4-[2-(trans-4-[(E)-3-methoxypropenyl]cyclohexyl)äthyl]benzol;
1-Chlor-4-[2-(trans-4-[(E)-3-methoxypropenyl]cyclohexyl)äthyl]benzol;
1-Chlor-2-fluor-4-[2-(trans-4-[(E)-3-methoxypropenyl]cyclohexyl)äthyl]benzol;
1,2-Difluor-4-[2-(trans-4-[(E)-3-methoxypropenyl]cyclohexyl)äthyl]benzol;
1-(Trifluormethyl)-4-[2-(trans-4-[(E)-3-methoxypropenyl]cyclohexyl)äthyl]benzol;
1-(Trifluormethoxy)-4-[2-(trans-4-[(E)-3-methoxypropenyl]yclohexyl)äthyl]benzol;
1-(Difluormethoxy)-4-[2-(trans-4-[(E)-3-methoxypropenyl]cyclohexyl)äthyl]benzol;
1-(Trifluoracetyl)-4-[2-(trans-4-[(E)-3-methoxypropenyl]cyclohexyl)äthyl]benzol;
1-Fluor-4-(2-[trans-4-(trans-4-[(E)-3-methoxypropenyl]cyclohexyl)cyclohexyl]äthyl)benzol;
1-Chlor-4-(2-[trans-4-(trans-4-[(E)-3-methoxypropenyl]cyclohexyl)cyclohexyl]äthyl)benzol;
1-Chlor-2-fluor-4-(2-[trans-4-(trans-4-[(E)-3-methoxypropenyl]cyclohexyl)cyclohexyl]äthyl)benzol;
1,2-Difluor-4-(2-[trans-4-(trans-4-[(E)-3-methoxypropenyl]cyclohexyl)cyclohexyl]äthyl)benzol;
1-(Trifluormethyl)-4-(2-[trans-4-(trans-4-[(E)-3-methoxypropenyl]cyclohexyl)cyclohexyl]äthyl)benzol;
1-(Trifluormethoxy)-4-(2-[trans-4-(trans-4-[(E)-3-methoxypropenyl]cyclohexyl)cyclohexyl]äthyl)benzol;
1-(Difluormethoxy)-4-(2-[trans-4-(trans-4-[(E)-3-methoxypropenyl]cyclohexyl)cyclohexyl]äthyl)benzol;
1-(Trifluoracetyl)-4-(2-[trans-4-(trans-4-[(E)-3-methoxypropenyl]cyclohexyl)cyclohexyl]äthyl)benzol;
1-Methoxy-2,3-difluor-4-[(trans-4-[(E)-3-methoxypropenyl]cyclohexyl)methoxy]benzol;
1-Aethoxy-2,3-difluor-4-[(trans-4-[(E)-3-methoxypropenyl]cyclohexyl)methoxy]benzol;
1-Propyloxy-2,3-difluor-4-[(trans-4-[(E)-3-methoxypropenyl]cyclohexyl)methoxy]benzol;
1-Butyloxy-2,3-difluor-4-[(trans-4-[(E)-3-methoxypropenyl]cyclohexyl)methoxy]benzol;
1-Methoxy-2,3-difluor-4-{[trans-4-(trans-4-[(E)-3-methoxypropenyl]cyclohexyl)cyclohexyl]methoxy}benzol;
trans-1-[trans-4-[(E)-3-Methoxypropenyl]cyclohexyl]-4-propylcyclohexan, Smp. (C-S_{B}) 32°C, S_{B}-N 70°C, Klp. (N-I) 73°C;
trans-1-[trans-4-[(E)-3-Aethoxypropenyl]cyclohexyl]-4-propylcyclohexan;
trans-1-[trans-4-[(E)-3-Propyloxypropenyl]cyclohexyl]-4-propylcyclohexan;
trans-1-[trans-4-[(E)-3-Methoxypropenyl]cyclohexyl]-4-pentylcyclohexan;
trans-1-[trans-4-[(E)-3-Aethoxypropenyl]cyclohexyl]-4-pentylcyclohexan;
trans-1-[trans-4-[(E)-3-Propyloxypropenyl]cyclohexyl]-4-pentylcyclohexan;
trans-1-[trans-4-[(E)-3-Methoxypropenyl]cyclohexyl]-4-vinylcyclohexan, Smp. (C-S_{B}) 9°C, S_{B}-N 18°C, Klp. (N-I) 36°C;
trans-1-[trans-4-[(E)-3-Methoxypropenyl]cyclohexyl]-4-[(E)-propenyl]cyclohexan, Smp. (C-N) 10°C, Klp. (N-I) 89°C;
trans-1-[trans-4-[(E)-3-Methoxypropenyl]cyclohexyl]-4-[3-butenyl]cyclohexan, Smp. (C-S_{B}) -17°C, S_{B}-N 67°C, Klp. (N-I) 75°C;
trans-1-[trans-4-[(E)-3-Methoxypropenyl]cyclohexyl]-4-[3E-pentenyl]cyclohexan, Smp. (C-S_{B}) 39°C, S_{B}-N 71°C, Klp. (N-I) 100°C;
trans-1-[trans-4-[(E)-3-Methoxypropenyl]cyclohexyl]-4-[4-pentenyl]cyclohexan, Smp. (C-S_{B}) 13°C, Klp. (S_{B}-I) 89°C;
trans-1-[trans-4-[(E)-3-Aethoxypropenyl]cyclohexyl]-4-[(E)-propenyl]cyclohexan;
trans-1-[trans-4-[(E)-3-Propyloxypropenyl]cyclohexyl]-4-[(E)-propenyl]cyclohexan;
4'-Chlor-4-[trans-4-[(E)-3-methoxypropenyl]cyclohexyl]biphenyl, Smp. (C-N) 140°C, Klp. (N-I) 208°C;
4'-Fluor-4-[trans-4-[(E)-3-methoxypropenyl]cyclohexyl]biphenyl, Smp. (C-N) 111°C, Klp. (N-I) 174°C;
4'-(Trifluormethyl)-4-[trans-4-[(E)-3-methoxypropenyl]cyclohexyl]biphenyl;
4'-(Trifluormethoxy)-4-[trans-4-[(E)-3-methoxypropenyl]cyclohexyl]biphenyl;
4'-(Difluormethoxy)-4-[trans-4-[(E)-3-methoxypropenyl]cyclohexyl]biphenyl;
4'-(Trifluoracetyl)-4-[trans-4-[(E)-3-methoxypropenyl]cyclohexyl]biphenyl;
4'-Chlor-3'-fluor-4-[trans-4-[(E)-3-methoxypropenyl]cyclohexyl]biphenyl, Smp. (C-N) 105°C, Klp. (N-I) 153°C;
3',4'-Difluor-4-[trans-4-[(E)-3-methoxypropenyl]cyclohexyl]biphenyl, Smp. (C-N) 91°C, Klp. (N-I) 107°C;
5-Propyl-2-[4-(trans-4-[(E)-3-methoxypropenyl]cyclohexyl)phenyl]pyrimidin;
5-Butyl-2-[4-(trans-4-[(E)-3-methoxypropenyl]cyclohexyl)phenyl]pyrimidin;
5-Pentyl-2-[4-(trans-4-[(E)-3-methoxypropenyl]cyclohexyl)phenyl]pyrimidin;
5-Hexyl-2-[4-(trans-4-[(E)-3-methoxypropenyl]cyclohexyl)phenyl]pyrimidin;
5-Heptyl-2-[4-(trans-4-[(E)-3-methoxypropenyl]cyclohexyl)phenyl]-Pyrimidin;
5-Propyl-2-[4-(trans-4-[(E)-3-methoxypropenyl]cyclohexyl)-phenyl]pyridin;
5-Butyl-2-[4-(trans-4-[(E)-3-methoxypropenyl]cyclohexyl)phenyl]pyridin;
5-Pentyl-2-[4-(trans-4-[(E)-3-methoxypropenyl]cyclohexyl)-phenyl]pyridin;
5-Hexyl-2-[4-(trans-4-[(E)-3-methoxypropenyl]cyclohexyl)phenyl]pyridin;
5-Heptyl-2-[4-(trans-4-[(E)-3-methoxypropenyl]cyclohexyl)-phenyl]pyridin.

### Beispiel 2

Ein Gemisch von 2,3 g 1-Brom-4-[trans-4-[(E)-3-methoxypropenyl]cyclohexyl]benzol, 1,7g Kupfer(I)cyanid und 45 ml 1-Methyl-2-pyrrolidon wurde 2,5 Stunden unter Argonbegasung bei 180°C erwärmt. Das abgekühlte Reaktionsgemisch wurde zu einer Lösung von 1,7 g Eisen(III)chlorid, 1 ml konzentrierter Salzsäure und 20 ml Wasser gegeben, 20 Minuten bei 55-60°C gerührt, abgekühlt und anschliessend dreimal mit je 50 ml Aethylacetat extrahiert. Die vereinigten organischen Phasen wurden zweimal mit je 100 ml konzentrierter Kochsalz-Lösung gewaschen, über Magnesiumsulfat getrocknet, filtriert und eingeengt. Der Rückstand wurde an Kieselgel mit Hexan/Aethylacetat (Vol. 9:1) chromatographisch gereinigt. Das erhaltene 4-[trans-4-[(E)-3-Methoxypropenyl]cyclohexyl]benzonitril wurde aus Hexan umkristallisiert. Dies ergab 0,8g reines Produkt mit Smp. (C-I) 66°C und Klp. (N-I) 59°C.

In analoger Weise können folgende Verbindungen hergestellt werden:
4-[trans-4-[(E)-3-Aethoxypropenyl]cyclohexyl]benzonitril;
4-[trans-4-[(E)-3-Propyloxypropenyl]cyclohexyl]benzonitril;
3-Fluor-4-[trans-4-[(E)-3-methoxypropenyl]cyclohexyl]benzonitril;
3-Fluor-4-[trans-4-[(E)-3-äthoxypropenyl]cyclohexyl]benzonitril;
3-Fluor-4-[trans-4-[(E)-3-propyloxypropenyl]cyclohexyl]benzonitril;
4-[trans-4-(trans-4-[(E)-3-Methoxypropenyl]cyclohexyl)cyclohexyl]benzonitril;
4-[trans-4-(trans-4-[(E)-3-Aethoxypropenyl]cyclohexyl)cyclohexyl]benzonitril;
4-(trans-4-(trans-4-[(E)-3-Propyloxypropenyl]cyclohexyl)cyclohexyl]benzonitril;
3-Fluor-4-[trans-4-(trans-4-[(E)-3-methoxypropenyl]cyclohexyl)cyclohexyl]benzonitril;
3-Fluor-4-[trans-4-(trans-4-[(E)-3-äthoxypropenyl]cyclohexyl)cyclohexyl]benzonitril;
3-Fluor-4-[trans-4-(trans-4-[(E)-3-propyloxypropenyl]cyclohexyl)cyclohexyl]benzonitril;
4-[2-(trans-4-[(E)-3-Methoxypropenyl]cyclohexyl)äthyl]benzol;
4-(2-[trans-4-(trans-4-[(E)-3-Methoxypropenyl]cyclohexyl)cyclohexyl]äthyl)benzol.

### Beispiel 3

0,6 g 4-[trans-4-[(E)-3-Methoxypropenyl]cyclohexyl]benzoesäure, 0,3 g 4-Hydroxybenzonitril und 0,04 g 4-(Dimethylamino)pyridin wurden in 50 ml Dichlormethan gelöst, die Lösung innert 10 Minuten unter Rühren portionenweise mit 0,6 g N,N'-Dicyclohexylcarbodiimid versetzt, das Gemisch über Nacht bei Raumtemperatur gerührt und dann filtriert. Das Filtrat wurde mit Dichlormethan verdünnt, zweimal mit je 50 ml gesättiger Natriumcarbonat-Lösung und einmal mit Wasser gewaschen, über Magnesiumsulfat getrocknet, filtriert und eingeengt. Das erhaltene Rohprodukt wurde an Kieselgel mit Hexan/ Aethylacetat (Vol. 9:1) chromatographisch gereinigt. Der 4-[trans-4-[(E)-3-Methoxypropenyl]cyclohexyl]benzoesäure-4-cyanophenylester wurde aus Methanol umkristallisiert. Dies ergab 0,6 g reines Produkt mit Smp. (C-N) 147°C und Klp. (N-I) 253°C.

Die als Ausgangsmaterial verwendete 4-[trans-4-[(E)-3-Methoxypropenyl]cyclohexyl]benzoesäure wurde wie folgt hergestellt:

Ein Gemisch aus 0,6 g 4-[trans-4-[(E)-3-Methoxypropenyl]cyclohexyl]benzonitril, 20 ml Aethylenglykol und 1,5 g Kaliumhydroxid wurde 3,5 Stunden auf 180°C erhitzt. Nach dem Erkalten wurde das Gemisch auf 300 ml Wasser gegossen und mit 3N Salzsäure angesäuert. Die ausgefallene 4-[trans-4-[(E)-3-Methoxypropenyl]cyclohexyl]benzoesäure wurde in Diäthyläther aufgenommen, mit Wasser neutral gewaschen, mit Natriumsulfat getrocknet und eingeengt. Umkristallisation des Rückstandes aus Aethanol ergab 0,4 g reines Produkt.

Auf analoge Weise können folgende Verbindungen hergestellt werden:
4-[trans-4-[(E)-3-Methoxypropenyl]cyclohexyl]benzoesäure-4-fluorphenylester, Smp. (C-N) 129°C, Klp. (N-I) 187°C;
4-[trans-4-[(E)-3-Methoxypropenyl]cyclohexyl]benzoesäure-4-chlorphenylester;
4-[trans-4-[(E)-3-Methoxypropenyl]cyclohexyl]benzoesäure-4-bromphenylester;
4-[trans-4-[(E)-3-Methoxypropenyl]cyclohexyl]benzoesäure-3,4-diflourphenylester, Smp. (C-N) 99°C, Klp. (N-I) 149°C;
4-[trans-4-[(E)-3-Methoxypropenyl]cyclohexyl]benzoesäure-4-(trifluormethoxy)phenylester, Smp. (C-N) 120°C, Klp. (N-I) 187°C;
4-[trans-4-[(E)-3-Methoxypropenyl]cyclohexyl]benzoesäure-2,3-difluor-4-äthoxyphenylester;
4-[trans-4-[(E)-3-Methoxypropenyl]cyclohexyl]benzoesäure-3-fluor-4-cyanophenylester;
4-[trans-4-[(E)-3-Methoxypropenyl]cyclohexyl]benzoesäure-4-methylphenylester;
4-[trans-4-[(E)-3-Methoxypropenyl]cyclohexyl]benzoesäure-4-äthylphenylester;
4-[trans-4-[(E)-3-Methoxypropenyl]cyclohexyl]benzoesäure-4-propylphenylester;
4-[trans-4-[(E)-3-Methoxypropenyl]cyclohexyl]benzoesäure-4-methoxyphenylester;
4-[trans-4-[(E)-3-Methoxypropenyl]cyclohexyl]benzoesäure-4-äthoxyphenylester;
4-[trans-4-[(E)-3-Methoxypropenyl]cyclohexyl]benzoesäure-4-cyanophenylester, Smp. (C-N) 147°C, Klp. (N-I) 253°C;
4-[trans-4-[(E)-3-Methoxypropenyl]cyclohexyl]benzoesäure-4-cyano-3-fluorophenylester, Smp. (C-N) 94°C, Klp. (N-I) 214°C.

### Beispiel 4

Die Eigenschaften der Verbindungen wurden untersucht, indem binäre Gemische mit 4-(trans-4-Pentylcyclohexyl)benzonitril hergestellt wurden, deren Schwellenspannung und Ansprechzeiten in einer TN-Zelle (low bias tilt) mit 8 »m Plattenabstand gemessen wurden; als Betriebsspannung wurde der 2,5-fache Wert der Schwellenspannung V₁₀ gewählt. Die entsprechenden Daten für reines 4-(trans-4-Pentylcyclohexyl)benzonitril betragen: Klp. (N-I) 54,6°C, V₁₀ = 1,62V, tₒₙ = 22ms, t_{off} = 40ms, Δn = 0,120.

### Mischung A

90 Gew.-% 4-(trans-4-Pentylcyclohexyl)benzonitril
10 Gew.-% 1-Fluor-4-[trans-4-(trans-4-[(E)-3-methoxypropenyl]cyclohexyl]cyclohexyl]benzol
Klp. (N-I) = 61°C, V₁₀ = 1,51V, tₒₙ = 36ms, t_{off} = 58ms, Δn = 0,121.

### Mischung B

80 Gew.-% 4-(trans-4-Pentylcyclohexyl)benzonitril
20 Gew.-% 1-Fluor-4-[trans-4-(trans-4-[(E)-3-methoxypropenyl]cyclohexyl]cyclohexyl]benzol
Klp. (N-I) = 67,3°C, V₁₀ = 1,56V, tₒₙ = 53ms, t_{off} = 89ms, Δn = 0,119.

### Mischung C

90 Gew.-% 4-(trans-4-Pentylcyclohexyl)benzonitril
10 Gew.-% 1,2-Difluor-4-[trans-4-(trans-4-[(E)-3-methoxypropenyl]cyclohexyl]cyclohexyl]benzol
Klp. (N-I) = 58,9°C, V₁₀ = 1,6V, tₒₙ = 33ms, t_{off} = 46ms, Δn = 0,121.

### Mischung D

80 Gew.-% 4-(trans-4-Pentylcyclohexyl)benzonitril
20 Gew.-% 1,2-Difluor-4-[trans-4-(trans-4-[(E)-3-methoxypropenyl]cyclohexyl]cyclohexyl]benzol
Klp. (N-I) = 63,8°C, V₁₀ = 1,86V, tₒₙ = 32ms, t_{off} = 47ms, Δn = 0,119.

## Patentansprüche

1. Verbindungen der allgemeinen Formel 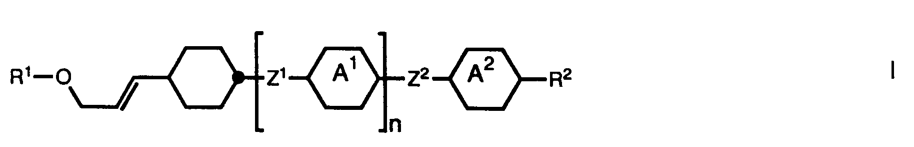 worin die Ringe A¹ und A² unabhängig voneinander unsubstituiertes oder mit Halogen substituiertes 1,4-Phenylen, in welchem falls unsubstituiert gegebenenfalls 1 oder 2 CH-Gruppen durch Stickstoff ersetzt sind, oder trans-1,4-Cyclohexylen oder trans-1,3-Dioxan-2,5-diyl darstellen; n entweder 0 oder 1 ist; Z¹ und Z² unabhängig voneinander eine einfache Kovalenzbindung oder -CH₂CH₂-, -COO-, -OOC-, -OCH₂-, -CH₂O-, -(CH₂)₄-, -O(CH₂)₃-, -(CH₂)₃O-, die trans-Form von -O-CH₂-CH=CH-, -CH=CH-CH₂-O-, -(CH₂)₂CH=CH- oder -CH=CH-(CH₂)₂-bedeuten; R¹ eine Alkylgruppe bezeichnet; R² Halogen, Cyano, Trifluoracetyl, gegebenenfalls mit Fluor substituiertes Alkyl mit 1 bis 12 Kohlenstoffatomen, in welchem gegebenenfalls 1 oder 2 nicht benachbarte -CH₂-Gruppen durch Sauerstoff und/oder eine -CH₂-CH₂- Gruppe durch -CH=CH-ersetzt sind, darstellt.

2. Verbindungen nach Anspruch 1, dadurch gekennzeichnet, dass R¹ einen geradkettigen Alkylrest mit 1 bis 7 Kohlenstoffatomen, vorzugsweise mit 1 bis 3 Kohlenstoffatomen, oder einen verzweigten, gegebenenfalls chiralen Alkylrest mit 3 bis 7 Kohlenstoffatomen darstellt.

3. Verbindungen nach Anspruch 1 oder 2, dadurch gekennzeichnet, dass eine der Gruppen Z¹ und Z² eine einfache Kovalenzbindung bedeutet.

4. Verbindungen nach einem der Ansprüche 1 bis 3, gekennzeichnet durch die allgemeine Formel 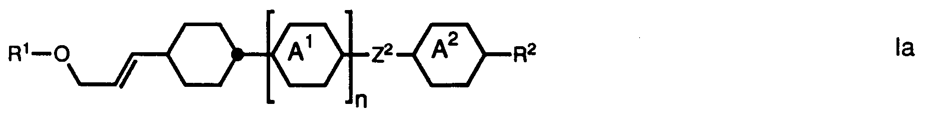 worin Ring A¹ 1,4-Phenylen oder trans-1,4-Cyclohexylen bedeutet; n entweder 0 oder 1 ist; Ring A² unsubstituiertes oder mit Halogen substituiertes 1,4-Phenylen bedeutet; R¹, R² und Z² die in Anspruch 1 angegebenen Bedeutungen haben.

5. Verbindungen nach einem der Ansprüche 1 bis 4, gekennzeichnet durch die allgemeine Formel 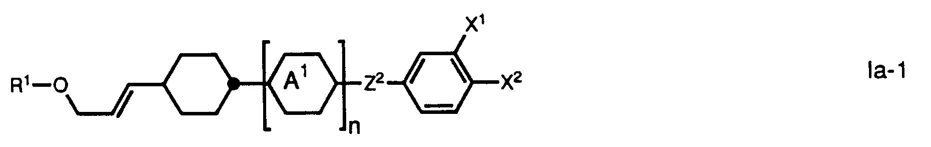 worin R¹ die in Formel I angegebene Bedeutung hat; n, Z² und Ring A¹ die in Formel Ia angegebenen Bedeutungen haben; X¹ Wasserstoff, Chlor oder Fluor bedeutet; und X² Fluor oder Chlor bezeichnet.

6. Verbindungen nach einem der Ansprüche 1 bis 4, gekennzeichnet durch die allgemeine Formel 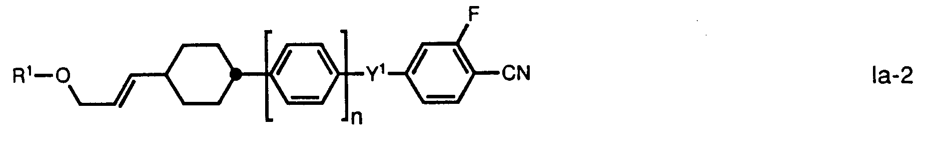 worin R¹ und n die in Formel I angegebene Bedeutung haben; Y¹ eine einfache Kovalenzbindung, -CH₂CH2- oder -COO- bezeichnet.

7. Verbindung nach einem der Ansprüche 1 bis 4, gekennzeichnet durch die allgemeine Formel 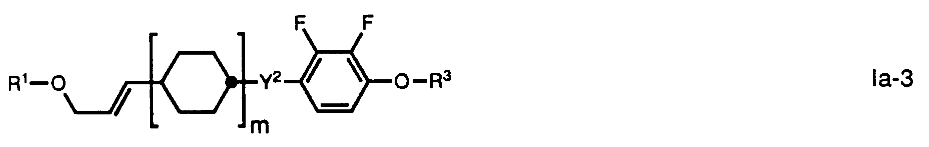 worin R¹ die in Formel I angegebene Bedeutung hat; m entweder 1 oder 2 ist; Y² eine einfache Kovalenzbindung, -CH₂-CH₂- oder -CH₂O- darstellt; und R³ eine Alkylgruppe mit 1-12 Kohlenstoffatomen, in welcher gegebenenfalls eine -CH₂CH2- Gruppe durch -CH=CH- ersetzt ist, oder eine Haloalkylgruppe bedeutet.

8. Verbindungen nach einem der Ansprüche 1 bis 3, gekennzeichnet durch die allgemeine Formel 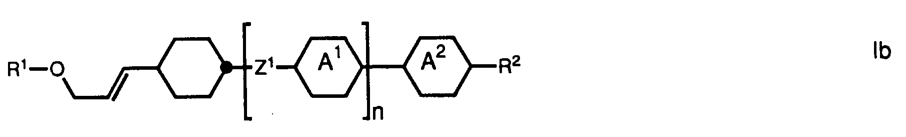 worin R¹ die in Formel I angegebene Bedeutung hat; Z¹ die in Formel I angegebenen Bedeutungen hat, vorzugsweise eine einfache Kovalenzbindung, -CH₂CH₂- oder -COO- darstellt; n entweder 0 oder 1 ist; R² und die Ringe A¹ und A² die in Formel I angegebenen Bedeutungen haben.

9. Verbindungen nach einem der Ansprüche 1 bis 3 und 8, gekennzeichnet durch die allgemeinen Formeln 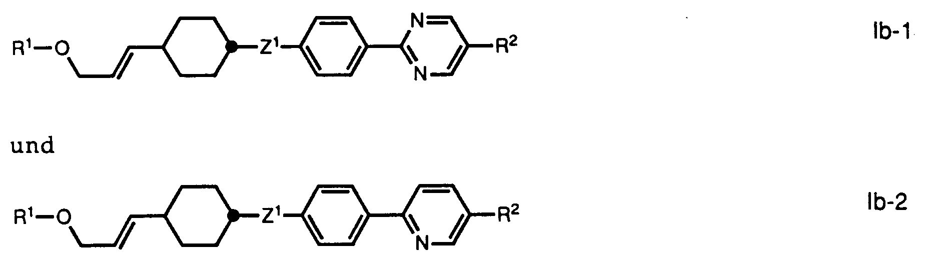 worin R¹ und R² die in Formel I gegebenen Bedeutungen haben; und Z¹ die in Formel I gegebene Bedeutung hat, vorzugsweise eine einfache Kovalenzbindung oder -COO- bezeichnet.

10. Flüssigkristallines Gemisch mit mindestens 2 Komponenten, dadurch gekennzeichnet, dass mindestens eine Komponente ein Verbindung der in Anspruch 1 definierten Formel I ist.

11. Verwendung der Verbindungen der in Anspruch 1 definierten Formel I für elektro-optische Zwecke.

## Claims

1. Compounds of the general formula 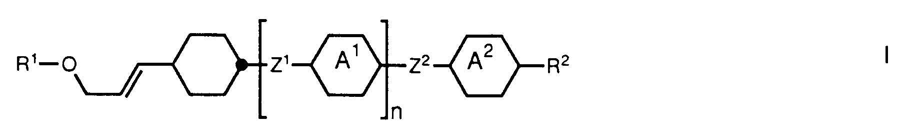 wherein rings A¹ and A² each independently represent unsubstituted or halogen-substituted 1,4-phenylene, in which optionally 1 CH group or 2 CH groups is/are replaced by nitrogen, or trans-1,4-cyclohexylene or trans-1,3-dioxane-2,5-diyl; n is either 0 or 1; Z¹ and Z ² each independently signify a single covalent bond or -CH₂CH₂-, -COO-, -OOC-, -OCH₂-, -CH₂O-, -(CH₂)₄-, -O(CH₂)₃-, -(CH₂)₃O-, the trans form of -O-CH₂-CH=CH-, -CH=CH-CH₂-O-, -(CH₂)₂CH=CH- or -CH=CH-(CH₂)₂-; R¹ denotes an alkyl group; R² represents halogen, cyano, trifluoroacetyl, optionally fluorine-substituted alkyl with 1 to 12 carbon atoms, in which optionally 1 -CH₂- group or 2 non-adjacent -CH₂- groups is/are replaced by oxygen and/or a -CH₂CH₂- group is replaced by -CH=CH-.

2. Compounds according to claim 1, characterized in that R¹ represents a straight-chain alkyl group with 1 to 7 carbon atoms, preferably with 1 to 3 carbon atoms, or a branched, optionally chiral, alkyl group with 3 to 7 carbon atoms.

3. Compounds according according to claim 1 or 2, characterized in that one of the groups Z¹ and Z² signifies a single covalent bond.

4. Compounds according to any one of claims 1 to 3, characterized by the general formula 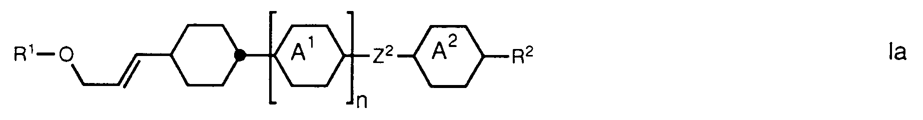 wherein ring A¹ signifies 1,4-phenylene or trans-1,4-cyclohexylene; n is either 0 or 1; ring A² signifies unsubstituted or halogen-substituted 1,4-phenylene; R¹, R² and Z² have the significances given in claim 1.

5. Compounds according to any one of claims 1 to 4, characterized by the general formula 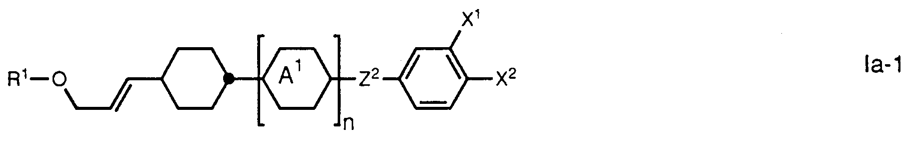 wherein R¹ has the significance given in formula I; n, Z² and ring A¹ have the significances given in formula Ia; X¹ signifies hydrogen, chlorine or fluorine; and X² denotes fluorine or chlorine.

6. Compounds according to any one of claims 1 to 4, characterized by the general formula 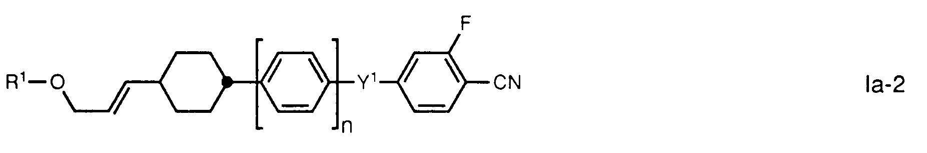 wherein R¹ and n have the significance given in formula I; Y¹ denotes a single covalent bond, -CH₂CH₂- or -COO-.

7. Compounds according to any one of claims 1 to 4, characterized by the general formula 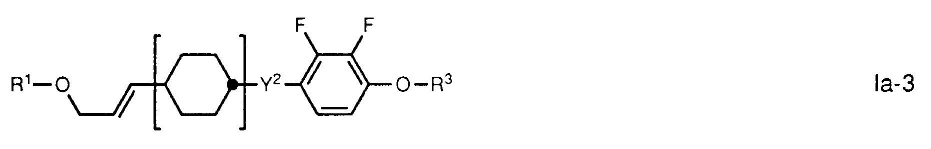 wherein R¹ has the significance given in formula I; m is either 1 or 2; Y² represents a single covalent bond, -CH₂CH₂- or -CH₂O-; and R³ signifies an alkyl group with 1-12 carbon atoms, in which optionally a -CH₂CH₂- group is replaced by -CH=CH-, or a haloalkyl group.

8. Compounds according to any one of claims 1 to 3, characterized by the general formula 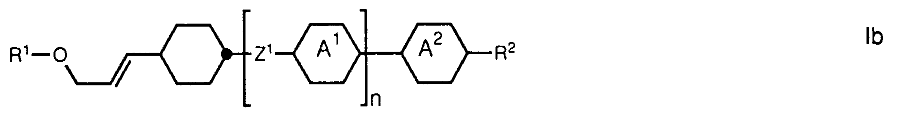 wherein R¹ has the significance given in formula I; Z¹ has the significances given in formula I, preferably a single covalent bond, -CH₂CH₂- or -COO-; n is either 0 or 1; R² and rings A¹ and A² have the significances given in formula I.

9. Compounds according to any one of claims 1 to 3 and 8, characterized by the general formulae 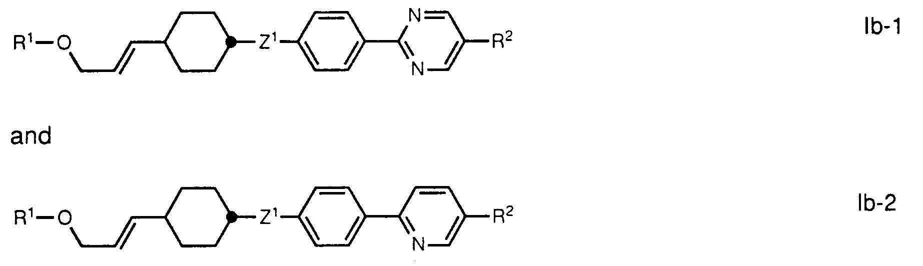 wherein R¹ and R² have the significances given in formula I; and Z¹ has the significance given in formula I, preferably a single covalent bond or -COO-.

10. A liquid crystalline mixture with at least 2 components, characterized in that at least one component is a compound of formula I defined in claim 1.

11. The use of the compounds of formula I defined in claim 1 for electro-optical purposes.

## Revendications

1. Composés de formule générale 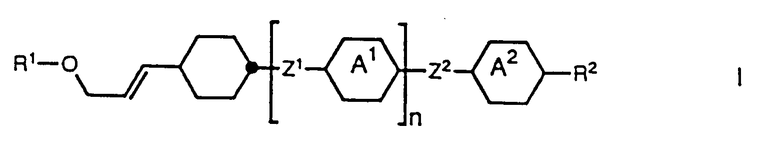 dans laquelle les cycles A¹ et A² représentent indépendamment l'un de l'autre un 1,4-phénylène non-substitué ou substitué par un halogène, dans lequel au cas où il est non-substitué le cas échéant 1 ou 2 groupes CH sont remplacés par l'azote, ou un trans-1,4-cyclohexylène, ou un trans-1,3-dioxan-2,5-diyle ; n est soit 0 soit 1 ; Z¹ et Z² représentent indépendamment l'un de l'autre une liaison de covalence simple ou -CH₂CH₂-, -COO-, -OOC-, -OCH₂-, -CH₂O-, -(CH₂)₄-, -O(CH₂)₃- -(CH₂)₃O-, la forme trans de -O-CH₂-CH=CH-, -CH=CH-CH₂-O-, -(CH₂)₂CH=CH- ou -CH=CH-(CH₂)₂- ;R¹ représente un groupe alkyle ; R² représente un halogène, un cyano, un trifluoroacétyle, un alkyle de 1 à 12 atomes de carbone substitué le cas échéant par un fluor, et dans lequel le cas échéant 1 ou 2 groupes -CH₂-non-voisins sont remplacés par l'oxygène et/ou un groupe -CH₂-CH₂- est remplacé par -CH=CH-.

2. Composés selon la revendication 1, caractérisés en ce que R¹ représente un reste alkyle à chaîne linéaire de 1 à 7 atomes de carbone, de préférence de 1 à 3 atomes de carbone, ou un reste alkyle ramifié le cas échéant chiral de 3 à 7 atomes de carbone.

3. Composés selon la revendication 1 ou 2, caractérisés en ce que l'un des groupes Z¹ et Z² représente une liaison de covalence simple.

4. Composés selon l'une des revendications 1 à 3, caractérisés par la formule générale 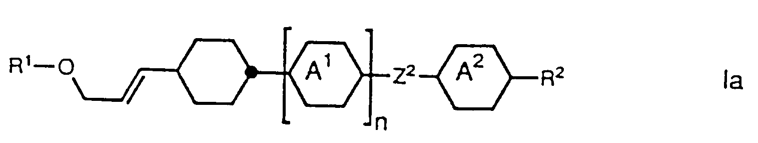 dans laquelle le cycle A¹ représente un 1,4-phénylène ou un trans-1,4-cyclohexylène ; n est soit 0 soit 1 ; le cycle A² représente un 1,4-phénylène non-substitué ou substitué par un halogène ; R¹, R² et Z² ont les significations données à la revendication 1.

5. Composés selon l'une des revendications 1 à 4, caractérisés par la formule générale 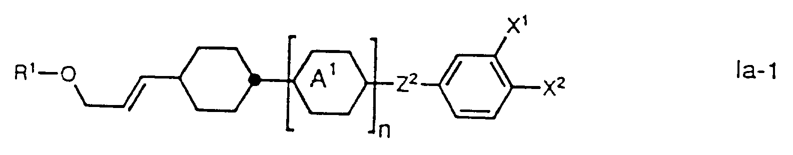 dans laquelle R¹ a la signification donnée pour la formule I ; n, Z² et le cycle A¹ ont les significations données à la formule Ia ; X¹ représente un hydrogène, un chlore ou un fluor ; et X² représente un fluor ou un chlore.

6. Composés selon l'une des revendications 1 à 4, caractérisés par la formule générale 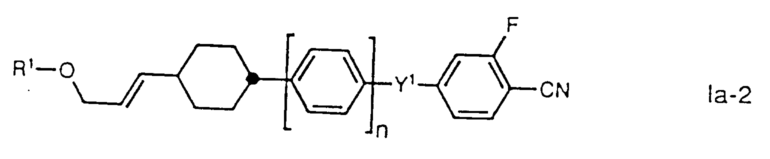 dans laquelle R¹ et n ont la signification donnée à la formule I ; Y¹ représente une liaison de covalence simple, -CH₂CH₂- ou -COO-.

7. Composés selon l'une des revendications 1 à 4, caractérisés par la formule générale 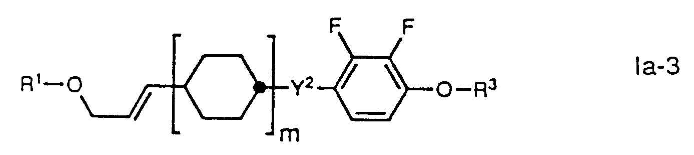 dans laquelle R¹ a la signification donnée pour la formule I ; m est soit 1 soit 2 ; Y² est une liaison de covalence simple, -CH₂-CH₂- ou -CH₂O- ; et R³ représente un groupe alkyle de 1-12 atomes de carbone, dans lequel le cas échéant un groupe -CH₂CH₂- est remplacé par -CH=CH-, ou représente un groupe haloalkyle.

8. Composés selon l'une des revendications 1 à 3, caractérisés par la formule générale 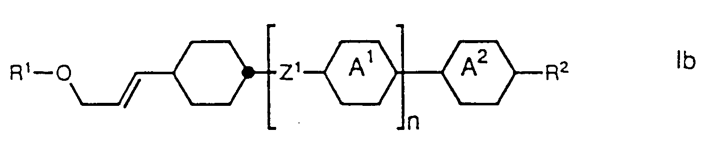 dans laquelle R¹ a la signification indiquée à la formule I ; Z¹ a les significations indiquées à la formule I, de préférence représente une liaison de covalence simple, -CH₂CH₂- ou -COO- ; n est soit 0 soit 1 ; R² et les cycles A¹ et A² ont les significations données pour la formule I.

9. Composés selon l'une des revendications 1 à 3 et 8, caractérisés par les formules générales 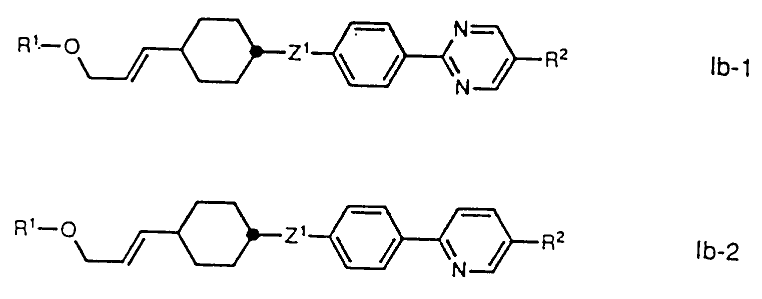 dans lesquelles R¹ et R² ont les significations données à la formule I ; et Z¹ a la signification donnée pour la formule I et représente de préférence une liaison de covalence simple ou -COO-.

10. Mélange de cristaux liquides ayant au moins deux composants, caractérisé en ce qu'au moins un composant est un composé de formule I défini à la revendication 1.

11. Utilisation des composés de formule I définis à la revendication 1 pour des utilisations électro-optiques.
